Europäisches Patentamt

European Patent Office

Office européen des brevets

(19)

(11) Veröffentlichungsnummer: **0 202 445 B1**

## (12) EUROPÄISCHE PATENTSCHRIFT

(45) Veröffentlichungstag der Patentschrift: 20.02.91

(51) Int. Cl.⁵: **A61K 9/22**

(21) Anmeldenummer: 86104723.1

(22) Anmeldetag: 07.04.86

(54) **Cytostatikhaltiges Pharmakadepot.**

(30) Priorität: 18.04.85 DE 3513938

(43) Veröffentlichungstag der Anmeldung:
26.11.86 Patentblatt 86/48

(45) Bekanntmachung des Hinweises auf die
Patenterteilung:
20.02.91 Patentblatt 91/08

(84) Benannte Vertragsstaaten:
AT BE CH DE FR GB IT LI NL SE

(56) Entgegenhaltungen:
EP-A- 0 016 906
DE-A- 2 511 122
DE-A- 2 651 441
DE-A- 2 727 535
DE-A- 3 037 270

CHEMICAL ABSTRACTS, Band 92, Nr. 13, 31.
März 1980, Seite 68, Ref.Nr. 104469a; Columbus, Ohio, USA K. SLAVIK et al.: "Localized
chemotherapy of solid experimental tumors
with macromolecular carriers containing antifolates."

CHEMICAL ABSTRACTS, Band 94, Nr. 5, 2.
Februar 1981, Seite 51, Ref.Nr. 25022n; Columbus, Ohio, USA G.A. SPECTOR et al.:

"Effect of gentamicin and irradiation on the
toxicity of high-dose methotrexate in rats."

(73) Patentinhaber: **MERCK PATENT GESELL-
SCHAFT MIT BESCHRÄNKTER HAFTUNG
Frankfurter Strasse 250 Postfach 4119
D-6100 Darmstadt(DE)**

(72) Erfinder: **Wahlig, Helmut, Dr.
Roemheldweg 16
D-6100 Darmstadt(DE)**
Erfinder: **Dingeldein, Elvira, Dr.
Am Spitzenpfad 9
D-6072 Dreieich(DE)**

## Beschreibung

Die Erfindung betrifft ein im Körper implantierbares Pharmakadepot zur kontrollierten verzögerten Freisetzung von Cytostatika.

Im Körper implantierbare Materialien, die Cytostatika enthalten, sind in großer Zahl bekannt. Als Basismaterialien sind sowohl organische Polymere wie z.B. Polyglycolide, Polylactide, Silikongummi, Polycarbonsäuren, Kollagen oder Gelatine genannt als auch anorganische Materialien wie z.B. gesintertes oder gepreßtes Tricalziumphosphat.

Die bisher vorgeschlagenen Materialien sollen in vorgefertigter Form geliefert und vom Chirurgen an geeigneter Stelle implantiert werden. Neben der Aufgabe als Wirkstoffdepot zu wirken, haben diese Materialien in der Regel keine weitere Funktion. In vielen Fällen wie z.B. bei der Sanierung von Knochendefekten ist es jedoch so, daß das Implantat auch stützende oder tragende Funktionen übernehmen muß. So z.B., wenn bei der Entfernung bösartig befallener Knochenteile die Stabilität des verbleibenden Knochens nicht mehr gewährleistet ist, oder wenn bei der Sanierung eines Defekts eine Prothese implantiert werden muß. In diesen Fällen ist es außerdem wesentlich, daß das Material dem Defekt optimal angepaßt werden kann.

Es bestand daher ein Bedürfnis nach einem Material, das sowohl in vorgefertigter Form geliefert werden kann als auch in einer Form zur Verfügung gestellt verden kann, die es dem Chirurgen erlaubt, das Material optimal den örtlichen Gegebenheiten anzupassen, das in der Lage ist, auch stützende und tragende Funktionen zu erfüllen und das darüber hinaus auch das inkorporierte Cytostatikum in zuverlässig reproduzierbarer Weise in wirksamen Mengen freisetzt.

Diese Aufgabe wurde durch die vorliegende Erfindung gelöst. Es wurde nämlich gefunden, daß ein an sich als knochenzement bekanntes Kunststoffmaterial auf Basis von Polyacrylaten und/oder Polymethacrylaten auch als Basismaterial für ein cytostatikumhaltiges Wirkstoffdepot geeignet ist und dabei alle Voraussetzungen in Bezug auf Festigkeit und Formgebung erfüllt, das aber auch in der Lage ist, das Cytostatikum in wirksamen Mengen freizusetzen, wenn dem Basismaterial eine Aminosäure zugemischt wird, insbesondere dann, wenn die Aminosäure in einer speziellen Korngröße eingesetzt wird.

Gegenstand der Erfindung ist daher ein im Körper implantierbares Pharmakadepot zur kontrollierten verzögerten Freisetzung von Cytostatika, das dadurch gekennzeichnet ist, daß ein Kunststoff auf Basis von Polyacrylaten und/oder Polymethacrylaten ein Cytostatikum und zumindest eine Aminosäure enthält.

Aus der DE-A- 2 727 535 und der EP-A-0 016 906 sind ähnliche Materialien bekannt. Im Unterschied zur vorliegenden Erfindung wird dort die Freisetzung von Antibiotika beschrieben. Über den Einfluß der Korngröße dabei verwendeter Aminosäuren ist in den beiden Dokumenten jedoch nichts ausgesagt.

Gegenstand der Erfindung ist auch ein Vorprodukt zur Herstellung eines solchen Pharmakadepots, das etwa 50 bis 75 Gew.% eines feinteiligen Polymerisats von Acryl und/oder Methacrylsäureestern, das gegebenenfalls weitere Zusätze wie z.B. Röntgenkontrastmittel, Farbstoffe und Katalysatoren enthalten kann, 1 bis 15 Gew.% einer Aminosäure, 0,1 bis 4 Gew.% eines Cytostatikums und 20 bis 45 Gew.% eines Acryl- und/oder Methacrylsäureestermonomeren enthält, das gegebenenfalls weitere Zusätze wie z.B. Stabilisatoren und Polymerisationsbeschleuniger enthält.

Gegenstand der Erfindung ist weiterhin ein Verfahren zur Herstellung eines solchen Pharmakadepots, das dadurch gekennzeichnet ist, daß man etwa 50 bis 75 Gewichtsteile eines feinteiligen Polymerisats von Acryl- und/oder Methacrylsäureestern, das gegebenenfalls weitere Zusätze wie z.B. Röntgenkontrastmittel, Farbstoffe und Katalysatoren enthalten kann, 1 bis 15 Gewichtsteile einer Aminosäure, 0,1 bis 4 Gewichtsteile eines Cytostatikums und 20 bis 45 Gewichtsteile eines Acryl- und/oder Methacrylsäureestermonomeren, das gegebenenfalls weitere Zusätze wie z.B. Stabilisatoren und Polymerisationsbeschleuniger enthält, zu einer halbfesten Paste vermengt, diese in eine gewünschte Form bringt und durch Polymerisation und Vernetzung aushärten läßt.

Gegenstand der Erfindung ist schließlich auf die Verwendung eines solchen Pharmakadepots bei der Bekämpfung von Tumoren.

Die Kunststoffmaterialien auf Basis von Polyacrylaten und/oder Polymethacrylaten, die als Ausgangsmaterialien zur Herstellung des erfindungsgemäßen Pharmakadepots eingesetzt werden, sind an sich bekannt. Sehr gebräuchlich ist z.B. ein Knochenzement, der in einer Normalpackung 2 Beutel mit je etwa 40 g Pulver und 2 Ampullen mit je 20 ml Flüssigkeit enthält. Das Pulver ist ein feines Perlpolymerisat aus Methacrylsäuremethylester mit einem Copolymeranteil von Acrylsäuremethylester.

Als Katalysator sind dem Pulver etwa 0,5 % Dibenzoylperoxid zugesetzt. Zur Kennzeichnung des Materials sind bei der Herstellung Spuren von Chlorophyll mit einpolymerisiert. Als Röntgenkontrastmittel kann das Pulver gegebenenfalls zusätzlich z.B. Zirkondioxid enthalten. Die zugehörige Flüssigkeit besteht aus monomerem Methacrylsäuremethylester, dem als Polymerisationsbeschleuniger etwa 0,7 % Dimethyl-p-toluidin sowie als Stabilisator Spuren von Hydrochinon zugesetzt sind. Auch diese Flüssigkeit ist in der Regel zur Kennzeichnung mit Spuren von Chlorophyll eingefärbt. Das in Polyethylenbeutel abgepackte Pulver ist mit Ethylenoxid sterilisiert. Die Flüssigkeit ist steril filtriert und in Glasampullen abgefüllt.

Beim Zusammenmischen von zwei Gewichtsteilen Pulver mit einem Gewichtsteil Flüssigkeit reagiert das Dibenzoylperoxid mit dem Dimethyl-p-toluidin in der Flüssigkeit, wodurch die radikalische Polymerisation angeregt wird. Die Mischung ist so abgestimmt, daß sie schon nach etwa einer Minute als teigige Paste verwendet werden kann. Diese Paste bleibt für mehrere Minuten knetbar und beginnt dann unter Wärmeentwicklung auszuhärten. Nach etwa 5 bis 10 Minuten ist die Polymerisation im wesentlichen abgeschlossen. Während der Polymerisation, solange die Paste noch formbar ist, kann diese in jede gewünschte Form gebracht werden, also z.B. zum Ausfüllen von Knochenhöhlen oder zum Einzementieren von Prothesen direkt in den Körper gebracht oder zur Herstellung von Formkörpern verwendet werden, die extrakorporal aushärten und danach an beliebigen Körperstellen eingesetzt werden können.

Erfindungsgemäß wird diesem Basismaterial ein Cytostatikum zugesetzt. Dieses kann als feinverteiltes Pulver den übrigen Bestandteilen, also dem Präpolymeren und dem Monomeren zugemischt und so in dem entstehenden Polymerisat homogen verteilt werden. Es kann aber auch bereits bei der Herstellung des Präpolymerisats in dieses eingearbeitet werden.

Cytostatika sind in großer Zahl bekannt und im Prinzip sind alle für den erfindungsgemäßen Zweck verwertbar, soweit sie mit den übrigen Bestandteilen des Depots verträglich sind und durch die beim Auspolymerisieren des Materials entstehende Wärme nicht zerstört werden. Vorzugsweise werden jedoch Adriamycin, 5-Fluoro-uracil und Methotrexat eingesetzt, wobei insbesondere mit Methotrexat sehr gute Ergebnisse erzielt werden.

Das Cytostatikum wird in wirksamen Mengen eingesetzt, die je nach dem verwendeten Wirkstoff unterschiedlich sein können. In der Regel wird eine solche Menge eingearbeitet, daß sich in dem Pharmakadepot eine Konzentration von etwa 0,1 bis 4 Gew.% ergibt. Bevorzugt sind insbesondere bei Methotrexat Konzentrationen von 0,2 bis 2 Gew.% und insbesondere von 0,4 bis 1 Gew.%.

Wesentlich für eine zuverlässige Freisetzung des Wirkstoffs ist die zusätzliche homogene Einarbeitung einer Aminosäure, wobei dabei relativ große Mengen von etwa 1 bis 15 Gew.% notwendig sind, die gewünschte Freisetzung des Wirkstoffs zu fördern. Bevorzugt werden etwa 2 bis 10 Gew.% und insbesondere etwa 3 bis 8 Gew.% eingesetzt. Geeignet sind im Prinzip alle natürlichen und sowohl mit dem Basismaterial als auch physiologisch verträglichen Aminosäuren, wobei insbesondere einbasische Aminosäuren mit Molekulargewichten von etwa 75 bis 200 in Betracht kommen wie z.B. Glycin, Alanin, Histidin, Leucin, Threonin und Arginin.

Besonders bevorzugt wird Arginin eingesetzt.

Als besonders überraschend hat sich herausgestellt, daß nicht nur die Menge der Aminosäure einen Einfluß auf die Freisetzung des Cytostatikums ausübt, sondern auch deren Korngröße. So wurde gefunden, daß insbesondere Korngrößen < 125 μm eine vorteilhaft gleichmäßige und reproduzierbare Freisetzung bewirken. Dazu können die handelsüblichen Materialien eingesetzt werden von denen Teilchen mit Durchmessern oberhalb 125 μm abgetrennt sind.

Es muß an dieser Stelle darauf hingewiesen werden, daß die Charakterisierung eines Teilchen durch die Teilchengröße bzw. den Durchmesser streng genommen nur bei Isometrischen Teilchen möglich ist, die also in allen drei Dimensionen gleiche Abmessungen besitzen (im Idealfall die Kugel). Dies ist bei dem handelsüblichen Material z.B. dem von E. Merck unter der Art.-Nr. 1542 erhältlichen Arginin auch der Fall. Überraschender Weise wurde jedoch festgestellt, daß die mit einem solchen Material erzielten guten Ergebnisse durch eine Veränderung der Form der Aminosäureteilchen noch verbessert werden können. So kann durch eine Mikronisierung der Teilchen, wobei ein stäbchenförmiges Material gewonnen wird, das nach der Kornanalyse durch Luftstrahlsiebung zu mindestens 95 Gew.% aus Teilchen < 50 μm besteht, eine weitere deutliche Verbesserung der Freisetzung erreicht werden.

In noch stärkerem Maße verbessert wird die Freisetzung des Wirkstoffs durch eine Lyophilisierung der Aminosäure. Dabei werden z.B. im Falle des Arginins nadelförmige Teilchen gewonnen, die ein Verhältnis von kleinster zu größter Dimension von etwa 1 zu 2 bis 1 zu 20 aufweisen. Die Länge dieser Teilchen kann bis zu mehreren 100 μm betragen, die Dicke ist jedoch in der Regel deutlich unter 50 μm.

Aus diesen Beispielen wird deutlich, daß eine zusätzliche Verbesserung der Wirkstofffreisetzung dadurch erreicht werden kann, daß die Aminosäureteilchen verkleinert und/oder in eine von der Kugelform weitgehend abweichende Form gebracht werden. Als fiktive "Durchmesser" eines solchen z.B. nadel- oder stäbchenförmigen Teilchens soll der eines kugelförmigen Teilchens gleichen Volumens verstanden werden. Im Sinne dieser Definition besitzt also ein stäbchenförmiges Teilchen mit einer Länge von etwa 500 μm und einer Dicke von etwa 50 μm ebenfalls einen "Durchmesser" von etwa 125 μm. Besonders bevorzugt sind Teilchen mit einem "Durchmesser" von unterhalb 50 μm und Stäbchen- oder Nadelform.

Die Zumischung der Aminosäure zu dem Basismaterial erfolgt analog der des Cytostatikums, wobei gegebenenfalls auch eine Vormischung von Aminosäure und Cytostatikum erfolgen kann oder die Aminosäure auch in das Präpolymere eingearbeitet werden kann.

Obwohl das erfindungsgemäße Pharmakadepot in erster Linie zur Bekämpfung von Tumoren eingesetzt wird, kann es doch vorteilhaft sein, dem Pharmakadepot zusätzlich noch Wirkstoffe anderer Wirkrichtungen beizufügen, insbesondere Antibiotika und/oder Antiseptika zur Bekämpfung oder Verhinderung von Infektionen am Implantationsort. Antibiotika sollten möglichst sowohl gegen grampositive als auch gramnegative Erreger wirksam sein und sollten bei den Erregern keine oder nur eine verzögerte Resistenz hervorrufen. Beispielsweise als geeignet können Aminoglycosidantibiotika wie Amikacin, Butirosin, Didesoxykanamycin B (DKP), Fortimycin, Gentamycin, Kanamycin, Lividomycin, Neomycin, Netilmicin, Ribostamycin, Sagamycine, Seldomycine und deren Epimere, Sisomicin, Sorbistin, Tobramycin, Streptomycine, Linkomycine wie Clindamycin, Lincomycin und Rifamycine wie Refampicin und Rifamycin genannt werden. Als Antiseptika kommen z.B. Bromchlorophen, Hexetidin, Buclosamid, Salicylsäure, Cernitrat, Chlorhexidin, 5-Chlor-8-hydroxy-chinolin, Kupfer-8-hydroxy-chinolat, Acridinorange, Undecensäure, Undecoyliumchlorid, Silbersalze wie Silbersulfadiazin, Mafenid, Nitrofurazol, Cloflucarban, Tribromsalan, Taurolin und Noxythiolin in Frage. Auch diese zusätzlichen Wirkstoffe, deren Art und Menge sich nach der gewünschten zusätzlichen Wirkung richtet, können den anderen Materialien auf an sich übliche Weise, vorzugsweise in feinverteilter Pulverform, zugemischt werden, wobei auch hier gegebenenfalls eine Vormischung mit einzelnen der anderen Bestandteilen oder eine Einarbeitung in das Präpolymerisat erfolgen kann.

Wie bereits erwähnt, kann das erfindungsgemäße Pharmakadepot fertig auspolymerisiert und damit in vorbestimmter Form zur Verfügung gestellt werden. Dies wird insbesondere dann sein, wenn z.B. beim Einsatz in Weichteilen lediglich die Funktion als lokale Wirkstoffquelle erfüllt werden muß. Dazu kann das Depot in beliebiger Form wie z.B. als Granulat, als Kubus, Kugel oder Ellipsoid, als Folie, Platte, Stift, Röhre oder anderer dem jeweiligen Einsatz angepaßter Form hergestellt werden.

In der Regel wird man dem Chirurg jedoch das erfindungsgemäße Material als Vorprodukt in die Hand geben, so daß die Formgebung erst bei der Implantation erfolgt und somit das Pharmakadepot den örtlichen Gegebenheiten optimal angepaßt und das Material auch bei der Implantation von Prothesen wie ein herkömmlicher Knochenzement eingesetzt werden kann.

Dazu werden die Bestandteile analog den oben beschriebenen bekannten Knochenzementen gebrauchsfertig so abgepackt, daß aufeinander abgestimmte Mengen der Feststoffe und der Flüssigkeit (des Monomeren) in einer Packung vorliegen. Aus diesem Vorprodukt kann dann in einfacher Weise das Pharmakadepot dadurch hergestellt werden, daß die Komponenten vermischt werden, wobei durch den der Feststoffkomponente beigefügten Katalysator die Polymerisation des Monomeren in Gang gesetzt wird und nach wenigen Minuten Reaktionszeit das ausgehärtete Endprodukt vorliegt. In der dazwischenliegenden Zeit in der das Material plastisch verformbar ist, kann es in den Körper eingebracht werden und dabei geformt werden.

Beispiel 1

39,2 g eines sterilen feinen Perlpolymerisats, bestehend aus einem Copolymerisat von Methylacrylat und Methylmethacrylat, das zusätzlich 0,5 % Dibenzoylperoxid und Spuren Chlorophyll enthält, werden mit 0,8 g L-Arginin (weitgehend isometrische Teilchen einer Größe < 125 µm) und 0,5 g Methotrexat vermischt und zusammen mit einer Ampulle mit 20 ml Methacrylsäuremethylester mit einem Zusatz von etwa 0,7 % Dimethyl-p-toluidin und etwa 0,006 % Hydrochinon (Monomerflüssigkeit) steril zu einer gebrauchsfertigen Einheit verpackt.

In den folgenden Beispielen wird analog verfahren, wobei folgende Mengen der Ausgangsmaterialien eingesetzt werden:

Beispiel 2

38,0 g Perlpolymerisat, 2,0 g L-Arginin, 0,5 g Methotrexat, 20,0 ml Monomerflüssigkeit

Beispiel 3

37,0 g Perlpolymerisat, 3,0 g L-Arginin, 0,5 g Methotrexat, 20,0 ml Monomerflüssigkeit

Beispiel 4

36,0 g Perlpolymerisat, 4,0 g L-Arginin, 0,5 g Methotrexat, 20,0 ml Monomerflüssigkeit

Beispiel 5

34,0 g Perlpolymerisat, 6,0 g L-Arginin, 0,5 g Methotrexat, 20,0 ml Monomerflüssigkeit

Beispiel 6

37,0 g Perlpolymerisat, 3,0 g L-Arginin, 0,1 g Methotrexat, 20,0 ml Monomerflüssigkeit

Beispiel 7

37,0 g Perlpolymerisat, 3,0 g L-Arginin, 0,25 g Methotrexat, 20,0 ml Monomerflüssigkeit

Beispiele 8 bis 14

Es wird analog den Beispielen 1 bis 7 verfahren, wobei das Arginin in mikronisierter Form mit einem Teilchendurchmesser von unter 50 μm eingesetzt wird.

Beispiele 15 bis 21

Es wird analog den Beispielen 1 bis 7 verfahren, wobei das Arginin in lyophilisierter Form (Nadeln) mit einem Teilchendurchmesser von unter 125 μm eingesetzt wird.

Beispiele 22 bis 42

Es wird analog den Beispielen 1 bis 7 verfahren, wobei anstelle von Arginin jeweils L-Histidin, L-Leucin oder L-Threonin eingesetzt wird.

Beispiele 43 bis 49

Es wird analog den Beispielen 1 bis 7 verfahren, wobei den Feststoffen zusätzlich 0,5 g Gentamycin zugemischt wird.

Beispiele 50 bis 56

Es wird analog den Beispielen 1 bis 7 verfahren, wobei den Feststoffen zusätzlich 0,25 g Gentamycin zugemischt wird.

Beispiel 57

Aus einem Vorprodukt nach Beispiel 4 wird durch Vermischen der Feststoffkomponente mit der Monomerflüssigkeit eine Paste bereitet, die man von Hand oder mit Hilfe einer Spritze in eine Knochenhöhle einbringt oder einpreßt und dort aushärten läßt.

In gleicher Weise können die Vorprodukte nach den übrigen der Beispiele 1 bis 56 eingesetzt werden.

In die in den Körper eingebrachte, noch nicht ausgehärtete Paste kann eine Prothese wie z.B. eine Endoprothese eingepreßt werden. Nach dem Aushärten des Kunststoffs ist die Prothese fest im Körper verankert.

Beispiel 58

36 g eines feinteiligen Copolymerisats aus Methylacrylat und Methylmethacrylat, das zusätzlich 0,5 % Dibenzoylperoxid und Spuren Chlorophyll enthält, 4 g mikronisiertes L-Arginin, 0,5 g Methotrexat, 0,5 g Gentamycin und 20 ml Methylmethacrylat, das etwa 0,7 % Dimethyl-p-toluidin und etwa 0,006 % Hydrochinon enthält, werden gründlich vermischt. Aus der entstehenden Paste werden Kugeln, Stifte, Ovoide sowie auch größere Implantate wie Zylinder, Röhren, Platten, Folien und andere Teile beliebiger Form und Größe geformt, die nach wenigen Minuten ausgehärtet sind. Die Teile werden steril verpackt und können als lokale Wirkstoffdepots eingesetzt werden.

In analoger Weise können Vorprodukte entsprechend den Beispielen 1 bis 56 zu Formkörpern verarbeitet werden.

**Ansprüche**

1. Im Körper implantierbares Pharmakadepot zur kontrollierten verzögerten Freisetzung von Cytostatika, dadurch gekennzeichnet, daß ein Kunststoff auf Basis von Polyacrylaten und/oder Polymethacrylaten ein Cytostatikum und zumindest eine Aminosäure mit einer Korngröße kleiner als 125μm enthält.

2. Pharmakadepot nach Anspruch 1, dadurch gekennzeichnet, daß das Cytostatikum in einer Menge von 0,1 bis 4 Gew. % enthalten ist.

3. Pharmakadepot nach Anspruch 1 oder 2, dadurch gekennzeichnet, daß die Aminosäure in einer Menge von 1 bis 15 Gew.% enthalten ist.

4. Pharmakadepot nach einem der Ansprüche 1 bis 3, dadurch gekennzeichnet, daß als Cytostatikum Methotrexat enthalten ist.

5. Pharmakadepot nach einem der Ansprüche 1 bis 4, dadurch gekennzeichnet, daß neben einem Cytostatikum noch ein Antibiotikum und/oder Antiseptikum enthalten ist.

6. Vorprodukt zur Herstellung eines Pharmakadepots nach Anspruch 1, enthaltend etwa 50 bis 75 Gew.% eines feinteiligen Polymerisats von Acryl- und/oder Methacrylsäureestern, das gegebenenfalls weitere Zusätze wie z.B. Röntgenkontrastmittel, Farbstoffe und Katalysatoren enthalten kann, 1 bis 15 Gew.% einer Aminosäure, 0,1 bis 4 Gew.% eines Cytostatikums und 20 bis 45 Gew.% eines Acryl- und/oder Methacrylsäureestermonomeren, das gegebenenfalls weitere Zusätze wie z.B. Stabilisatoren und Polymerisationsbeschleuniger enthält.

7. Vorprodukt nach Anspruch 6, dadurch gekennzeichnet, daß zusätzlich ein Antibiotikum und/oder ein Antiseptikum enthalten ist.

8. Verfahren zur Herstellung eines Pharmakadepots nach Anspruch 1, dadurch gekennzeichnet, daß man etwa 50 bis 75 Gewichtsteile eines feinteiligen Polymerisats von Acryl- und/oder Methacrylsäureestern, das gegebenenfalls weitere Zusätze wie z.B. Röntgenkontrastmittel, Farbstoffe und Katalysatoren enthalten kann, 1 bis 15 Gewichtsteile einer Aminosäure, 0,1 bis 4 Gewichtsteile eines Cytostatikums und 20 bis 45 Gewichtsteile eines Acryl- und/oder Methacrylsäureestermonomeren, das gegebenenfalls weitere Zusätze wie z.B. Stabilisatoren und Polymerisationsbeschleuniger enthält, zu einer halbfesten Paste vermengt, diese in eine gewünschte Form bringt und durch Polymerisation und Vernetzung aushärten läßt.

9. Verfahren nach Anspruch 8, dadurch gekennzeichnet, das man in die Paste zusätzlich ein Antibiotikum und/oder ein Antiseptikum einarbeitet.

## Claims

1. Drug depot, which can be implanted in the body, for the controlled, delayed release of cytostatics, characterized in that a synthetic material based on polyacrylates and/or polymethacrylates contains a cytostatic and at least one amino acid having a particle size smaller than 125 μm.

2. Drug depot according to claim 1, characterized in that the cytostatic is present in an amount of 0.1 to 4% by weight.

3. Drug depot according to claims 1 or 2, characterized in that the amino acid is present in an amount of 1 to 15% by weight.

4. Drug depot according to one of claims 1 to 3 characterized in that the cytostatic present is methotrexate.

5. Drug depot according to one of claims 1 to 4 characterized in that an antibiotic and/or antiseptic is also present in addition to a cytostatic.

6. Precursor for the preparation of a drug depot according to claim 1, containing about 50 to 75% by weight of a finely divided polymer of acrylic and/or methacrylic esters, which can optionally contain further additives such as, for example, X-ray contrast media, pigments and catalysts, and containing 1 to 15% by weight of an amino acid, 0.1 to 4% by weight of a cytostatic, and 20 to 45% by weight of an acrylic and/or methacrylic ester monomer which optionally contains further additives such as, for example, stabilizers and polymerization accelerators.

7. Precursor according to claims 6 characterized in that an antibiotic and/or an antiseptic are additionally present.

8. Process for the preparation of a drug depot according to claim 1, characterized in that about 50 to 75 parts by weight of a finely divided polymer of acrylic and/or methacrylic esters, which can optionally contain further additives such as, for example, X-ray contrast media, pigments and catalysts, and 1 to 15 parts by weight of an amino acid, 0.1 to 4 parts by weight of a cytostatic, and 20 to 45 parts by weight of an acrylic and/or methacrylic ester monomer which optionally contains further additives such as, for example, stabilizers and polymerization accelerators are mixed to form a semi-solid paste, and the latter is converted into a desired shape and allowed to harden by polymerization and cross-linking.

9. Process according to claim 8, characterized in that an antibiotic and/or an antiseptic are additionally incorporated into the paste.

## Revendications

1. Dépôt pharmaceutique implantable dans le corps pour une libération contrôlée, avec retard, d'agents cytostatiques, caractérisé en ce qu' une matière plastique à base de polyacrylates et/ou de polyméthacrylates contient un agent cytostatique, et au moins un acide aminé dont la dimension des particules est inférieure à 125 μm.

2. Dépôt pharmaceutique selon la revendication 1, caractérisé en ce que l'agent cytostatique est contenu en quantité de 0,1 à 4 % en poids.

3. Dépôt pharmaceutique selon la revendication 1 ou la revendication 2, caractérisé en ce que l'acide aminé est contenu en quantité de 1 à 15 % en poids.

4. Dépôt pharmaceutique selon l'une des revendications 1 à 3, caractérisé en ce que l'agent cytostatique contenu est le méthotrexat.

5. Dépôt pharmaceutique selon l'une des revendications 1 à 4, caractérisé en ce que, en plus d'un agent cytostatique, il se trouve contenu un antibiotique et/ou un antiseptique.

6. Avant-produit pour préparer un dépôt pharmaceutique selon la revendication 1, comportant environ de 50 à. 75 % en poids d'un polymère à fines particules d'esters de l'acide acrylique et/ou de l'acide méthacrylique, qui peut éventuellement comporter d'autres additifs comme par exemple des agents de contraste pour rayons X, des colorants et des catalyseurs, de 1 à 15 % en poids d'un acide aminé, de 0,1 à 4 % en poids d'un agent cytostatique, et de 20 à 45 % en poids d'un ester monomère de l'acide acrylique et/ou de l'acide méthacrylique, qui comporte éventuellement d'autres additifs, comme par exemple des stabilisants et des accélérateurs de polymérisation.

7. Avant-produit selon la revendication 6, caractérisé en ce que, en outre, il se trouve contenu un antibiotique et/ou un antiseptique.

8. Procédé pour préparer un dépôt pharmaceutique selon la revendication 1, caractérisé en ce que l'on mélange, pour obtenir une pâte à moitié solide, environ de 50 à 75 parties en poids d'un polymère à fines particules d'esters de l'acide acrylique et/ou de l'acide méthacrylique, qui peut contenir éventuellement d'autres additifs, comme par exemple des agents de contraste pour rayons X, des colorants et des catalyseurs, de 1 à 15 parties en poids d'un acide aminé, de 0,1 à 4 parties en poids d'un agent cytostatique, et de 20 à 45 parties en poids d'un ester monomère de l'acide acrylique et/ou de l'acide méthacrylique, qui comporte éventuellement d'autres additifs, comme par exemple des stabilisants et des accélérateurs de polymérisation, en ce que l'on donne à la pâte la forme désirée, et en ce que l'on la laisse durcir-par polymérisation et réticulation.

9. Procédé selon la revendication 8, caractérisé en ce que l'on incorpore en plus dans la pâte un antibiotique et/ou un antiseptique.